Europäisches Patentamt

European Patent Office

Office européen des brevets

(11) Numéro de publication: **0 229 561 B1**

(12) **FASCICULE DE BREVET EUROPEEN**

(45) Date de publication de fascicule du brevet: **05.08.92**

(51) Int. Cl.5: **A61K 9/50**, A61K 7/00, A61K 31/07

(21) Numéro de dépôt: **86402756.0**

(22) Date de dépôt: **10.12.86**

Demande divisionnaire 91117890.3 déposée le 10/12/86.

(54) Composition pharmaceutique, notamment dermatologique, ou cosmétique, à base de phases lamellaires lipidiques hydratées ou de liposomes contenant un rétinoïde ou un analogue structural dudit rétinoïde tel qu'un caroténoîde.

(30) Priorité: **11.12.85 FR 8518362**

(43) Date de publication de la demande:
**22.07.87 Bulletin 87/30**

(45) Mention de la délivrance du brevet:
**05.08.92 Bulletin 92/32**

(84) Etats contractants désignés:
**AT BE CH DE FR GB IT LI LU NL SE**

(56) Documents cités:
**WO-A-82/02833**
**WO-A-85/03225**

**CHEMICAL ABSTRACTS, vol. 88, no. 19, 8 mai 1978, page 171, résumé no 132362w, Columbus, Ohio, US; H.Y. YAMAMOTO et al.: "Carotenoid organization in membranes; Thermal transition and spectral properties of carotenoid-containing liposomes", & BIOCHIM. BIOPHYS. ACTA 1978, 507(1), 119-27**

(73) Titulaire: **LVMH RECHERCHE**
**48-50, rue de Seine**
**F-92703 Colombes Cédex(FR)**

(72) Inventeur: **Meybeck, Alain**
**20ter, rue de Bezons**
**F-92400 Courbevoie(FR)**
Inventeur: **Michelon, Philippe**
**8 rue Valentin Häury**
**F-75015 Paris(FR)**
Inventeur: **Montastier, Christiane**
**10 rue Marguerite**
**F-78600 Maisons-Lafitte(FR)**
Inventeur: **Redziniak, Gérard**
**101 rue des Fauvettes**
**F-45590 Saint-Cyr-en-Val(FR)**

(74) Mandataire: **Durand, Yves Armand Louis et al Cabinet Z. Weinstein 20, Avenue de Friedland**
**F-75008 Paris(FR)**

Rank Xerox (UK) Business Services

CHEMICAL ABSTRACTS, vol. 101, no. 13, 24 septembre 1984, page 246, résumé no. 106051m, Columbus, Ohio, US; S.S. BRODY: "Liposomes containing various carotenoids and chlorophyll: temperature-induced changes in their spectral properties", & PHOTO-BIOCHEM. PHOTOBIOPHYS. 1984, 7(4), 205-19

"Les liposomes - Applications thérapeutiques" F. Puisieux et J. Delattre, pages 297-312, Ed. Tec et Doc (Janvier 1985)

## Description

La présente invention concerne des compositions pharmaceutiques, notamment dermatologiques, ou cosmétiques, à base de phases lamellaires lipidiques hydratées ou de liposomes contenant un rétinoïde ou un analogue structural dudit rétinoïde tel qu'un caroténoïde.

Il est bien connu que certains rétinoïdes, tels que la trétinoïne,sont utilisés en thérapeutique, en particulier dans le traitement contre l'acné.

La trétinoïne ou ses homologues, tels que l'acide 13-cis-rétinoïque ou l'étrétinate constituent des médicaments les plus utilisés à l'heure actuelle dans le traitement de l'acné. On connait également l'action anti-acnéique des caroténoïdes. Diverses formes galéniques ont été proposées parmi lesquelles la plus active est basée sur une solution alcoolique de la trétinoïne destinée à l'administration locale. On peut citer à ce propos un médicament ayant la dénomination commerciale ABEREL®. Il existe diverses formulations de ABEREL à 0,025%, 0,05% et 0,1% de trétinoïne. L'excipient est à base d'alcool éthylique à 95°. Les effets de la trétinoïne sont résumés dans le dictionnaire VIDAL, "Spécialités Pharmaceutiques Françaises", notamment dans l'édition 1985, et des commentaires plus complets peuvent être obtenus à partir de l'article de Lorraine Kligman et al publié dans "the Journal of investigative Dermatology, volume 73, no. 5, partie I, pages 354-358, publié en 1979, avec une expérimentation sur la peau de souris Hairless Rhino qui est le modèle le plus approprié pour l'expérimentation relative à l'inhibition des comédons. Dans cet article Kligman souligne que la trétinoïne a de plus un effet positif pour faire disparaître les plis et rides de la peau de la souris Hairless rhino.

Il existe également des médicaments destinés à l'administration orale contenant des rétinoïdes tels que l'acide 13-cis-rétinoïque ou l'étrétinate, pour le traitement des acnés graves, du psoriasis et autres troubles sérieux de la kératinisation.

L'inconvénient majeur de la trétinoïne est, par voie générale, sa toxicité systémique (tératogénicité, et des effets indésirables dûs à l'hypervitaminose A), et par voie locale, son action irritante pouvant nécessiter dans certains cas l'arrêt du traitement. On souligne dans le Dictionnaire VIDAL que cette action caractérisée par un érythème sec, légèrement cuisant, siégeant surtout au niveau de la région péribuccale et du cou, est directement liée à l'activité du produit, mais qu'elle disparaît toutefois lorsque l'on espace les applications.

D'après certains auteurs, la toxicité systémique de la trétinoïne, même par voie locale, est un facteur limitant pour son utilisation (voir avant dernier paragraphe page 357 de l'article susmentionné).

Ainsi, il apparait que l'effet irritant de la trétinoïne est lié directement à l'activité de celle-ci.

On peut observer en outre que l'on indique que les formes ABEREL ®, en excipients classiques, en solution à 0,2% et 0,3%, qui sont fortement dosées en trétinoïne, sont contre indiquées dans l'acné et réservées au traitement des troubles de la kératinisation.

Enfin, la solution alcoolique utilisée dans la forme ABEREL ® dosée à 0,025% s'avère aussi présenter un effet irritant.

Dans une autre application thérapeutique, on peut citer le brevet US Albert Kligman N° 3 856 934 relatif à une composition pour la dépigmentation de la peau contenant de la trétinoïne (également dénommée vitamine A acide ou encore acide trans-rétinoïque).

Lorraine Kligman et al. décrivent encore dans la revue "Connective tissue research, 1984, volume 12, pages 139-150 que la trétinoïne à une dose de 0,05%, appliquée pendant 5 et 10 semaines, stimule les fibroblastes et favorise la réparation des dommages causés par une irradiation ultraviolette. Ainsi, Kligman révèle dans cet article que la trétinoïne a une activité favorisant la formation d'une zone de reconstruction subépidermique, ce qui est considéré comme étant favorable pour "rajeunir" l'épiderme.

Par ailleurs, Yamamoto et autres dans Chem. Abs. vol. 88, N° 19, 8 Mai 1978, p. 171 et dans Biochim. Biophys. Acta 1978, 507(1), 119-27, ainsi que S.S. Brody et autres dans Chem. Abs., vol. 101, N° 13, 24 Septembre 1984, p. 246 et dans Photo biochem. Photobiophys. 1984, 7(4), 205-19, décrivent des liposomes pouvant contenir notamment divers caroténoïdes, mais ces liposomes ne sont pas décrits comme pouvant constituer des compositions pharmaceutiques, notamment dermatologique ou cosmétique.

Enfin dans un livre de F. Puisieux et S. Delattre intitulé "Les liposomes - Applications Thérapeutiques", Technique et Documentation Lavoisier, 1985, pages 297-312, il est décrit que l'on peut encapsuler dans des niosomes des composés liposolubles qui s'insèrent dans les feuillets lipidiques. Mais, en ce qui concerne les applications thérapeutiques des niosomes, il est seulement décrit que les niosomes permettent de prévenir le phénomène de peau sèche induit par des agressions diverses.

Ainsi, la présente invention a pour but de résoudre le nouveau problème technique consistant en la fourniture d'une nouvelle formulation thérapeutique d'agents actifs comprenant des rétinoïdes, caroténoïdes ou des composés à structure chimique analogue, permettant de potentialiser leur efficacité tout en diminuant leurs effets toxiques, notamment l'effet irritant sur la peau lors de l'application locale de certains.

La présente invention permet pour la première fois de résoudre ce nouveau problème technique dans le cadre d'un certain nombre d'applications thérapeutiques particulières.

Ainsi, la présente invention a pour objet l'utiltsation d'une composition à base de phases lamellaires lipidiques hydratées ou de liposomes qui contiennent à titre d'agent actif un rétinoïde ou un caroténoïde, pour l'obtention d'une composition pharmaceutique, notamment dermatologique, ou cosmétique destinée au traitement par application topique des différentes formes d'acné, de l'hyperkératose, du psoriasis, à la lutte contre le vieillissement cutané actinique ou chronologique, à la suppression ou l'atténuation des taches pigmentaires cutanées, à favoriser la cicatrisation, à la reconstitution des micro-vaisseaux sanguins, notamment dans le derme, et à la stimulation de la pousse des cheveux, ou destinée à l'administration orale, en particulier pour le traitement des différentes formes d'acné, l'hyperkératose et le psoriasis.

Selon un mode de réalisation particulier, le rétinoïde est avantageusement choisi parmi la trétinoïne ou ses dérivés notamment, sels ou esters ; l'acide 13-cis-rétinoïque ou ses dérivés, notamment sels ou esters ; la vitamine A ou ses esters tels que acétate, propionate, palmitate ou stéarate ; l'étrétinate. D'autre part le caroténoïde précité est de préférence choisi parmi le groupe consistant de l'$\alpha$ ou $\beta$-carotène, le $\gamma$-carotène ou le $\delta$-carotène.

Selon un mode de réalisation préféré, la teneur en poids de la trétinoïne relativement à la composition totale est de $10^{-6}$ à $10^{-3}$, et de préférence de $5.10^{-6}$ à $10^{-4}$.

Naturellement, les phases lamellaires lipidiques hydratées ou les liposomes précités peuvent également contenir d'autres substances hydrophobes telles que des stérols (sistostérol, cholestérol, stigmastérol, etc...), comme cela est habituel et a été décrit dans FR-A-2 521 565.

On peut combiner l'agent actif précité avec d'autres agents thérapeutiquement, dermatologiquement ou cosmétiquement actifs. Ainsi, il est possible de combiner cet agent actif avec un agent actif complémentaire ou secondaire incorporé soit dans ladite couche lipidique du liposome, et dans ce cas sa quantité ne devra pas excéder 40% en poids de la totalité de la phase lipidique, soit dans la phase aqueuse, en fonction de leur solubilité. Cet agent actif complémentaire peut être en particulier choisi parmi l'érythromycine, la clindamycine, le métronidazole, ou un autre agent antibiotique ou antiseptique où les dérivés des composés précités, notamment les esters. Dans ce cas, la teneur en cet agent actif complémentaire peut atteindre 4% pour l'érythromycine, 2% pour la clindamycine ou 2% pour le métronidazole, en poids par rapport à la composition totale. Ainsi, les concentrations habituelles en ces agents actifs complémentaires peuvent être utilisées en combinaison avec l'agent actif principal précité. Une combinaison particulièrement efficace consiste en une concentration de $5.10^{-5}$ en trétinoïne avec 1% d'érythromycine.

Il est également avantageux, et dans certains cas très importants, d'ajouter à la phase lipidique un antioxydant tel que l'$\alpha$-tocophérol jusqu'au plus 1% en poids, le B.H.T. ou le TBHQ. Il est en outre encore avantageux d'ajouter à la phase aqueuse des antioxydants hydrosolubles tels que l'acide ascorbique, le sulfite de sodium, le bisulfite de sodium, l'EDTA et des stablisants tels qu'acide citrique, histidine dans les proportions habituelles de leur utilisation. On peut aussi prévoir la présence d'un composé constituant un filtre ultra-violet.

De préférence, la proportion du rétinoïde ou de son analogue structural comme le caroténoïde, dans la phase lipidique constituant la bicouche des phases lamellaires ou liposomes n'est pas critique et peut atteindre 40% en poids. De préférence, cette proportion est comprise en $10^{-4}$% et 10%, encore de préférence entre 0,01% et 5%.

Les phases lamellaires lipidiques hydratées ou liposomes selon l'invention contenant un ou plusieurs des divers constituants cités plus haut, peuvent être avantageusement soumis à une lyophilisation, selon les techniques habituelles, pour donner une poudre lipidique. Ladite poudre, qui peut être conservée longtemps dans cet état sans aucune altération, est dispersée dans un milieu aqueux approprié, en vue de son utilisation, afin de préparer une composition pharmaceutique, notamment dermatologique, ou cosmétique selon l'invention.

Cette composition pharmaceutique, dermatologique ou cosmétologique selon l'invention peut être formulée pour traiter les différentes formes d'acné, l'hyperkératose, le psoriasis, et autres troubles de la kératinisation, pour lutter contre le vieillissement cutané actinique dû par exemple à des expositions répétées au soleil, contre le vieillissement cutané chronologique, pour atténuer ou supprimer les taches pigmentaires cutanées, pour favoriser une cicatrisation, pour la reconstitution des micro-vaisseaux sanguins notamment dans le derme et pour favoriser la stimulation de la pousse des cheveux. Cette composition peut également être préparée sous une forme appropriée pour l'administration orale, en particulier pour le traitement des différentes formes d'acné, l'hyperkératose, le psoriasis.

D'autres buts, caractéristiques et avantages de l'invention apparaîtront à la lumière de la description explicative qui va suivre donnant plusieurs exemples donnés simplement à titre d'illustration et qui ne sauraient donc en aucune façon limiter la portée de l'invention, et rapportant les résultats des évaluations d'activité, d'hyperacanthose et d'irritation. La figure annexée représente l'activité comédolytique en fonction de la concentration en vitamine A acide soit contenue dans les liposomes (courbe L-o-o-o) soit dans l'alcool (courbe-A-Δ-Δ-Δ). Les pourcentages sont donnés en poids sauf indication contraire.

**Exemple 1.**

Préparation de suspensions de liposomes contenant de la trétinoïne.

On dissout dans 30 ml de dichlorométhane, 2 g de lécithine de soja, 0,1g de trétinoïne en présence d'un antioxydant lipophile, par exemple 0,006g d'$\alpha$ tocophérol.

La solution obtenue peut être traitée pour obtenir une suspension de liposomes par le procédé d'évaporation en récipient rotatif bien connu et qui consiste à déposer une couche lipidique dans le ballon de l'évaporateur rotatif par évaporation du solvant, puis ajouter de l'eau ou une solution aqueuse appropriée.

On peut également utiliser la technique dite de la phase inverse décrite dans le document FR-A-2 399242.

Cependant, on préfère atomiser la solution obtenue à 65°C, comme décrit dans le document FR-A-2521565 de manière à produire une fine poudre que l'on disperse dans 200 ml d'une solution aqueuse tamponnée à pH 7,5 environ. Généralement on utilise une solution contenant 0,8% en NaCl et 1,5% $NaH_2PO_4$, dite "tampon phosphate", additionnée d'un stabilisateur antioxydant tel que de l'acide ascorbique à la concentration de 0,05%.

On obtient ainsi, après homogénéisation soit aux ultra-sons, soit dans un homogénéisateur sous pression, par exemple selon le procédé décrit dans le document FR-A-2 534 487, ladite suspension de liposomes dans laquelle la concentration en trétinoïne est d'environ $5.10^{-4}$ et la concentration en lécithine est de 1%.

On observera qu'on peut ainsi réaliser diverses dilutions en modifiant la quantité de trétinoïne ajoutée au départ ou en augmentant le volume de la solution de dispersion, ce qui constitue un procédé aisé de préparation de diverses concentrations en trétinoïne.

**Exemple 2.**

Utilisation de compositions conformes à l'exemple 1 pour constituer une composition pharmaceutique ou cosmétique.

On vérifie l'utilisation de la composition de l'exemple 1 à titre de composition pharmaceutique ou cosmétique en effectuant les expérimentations in vivo suivantes, sur la souris hairless Rhino.

A - Activité comédolytique

Pour étudier l'activité comédolytique sur la souris hairless Rhino, on utilise des suspensions de liposomes à 3% en lécithine et différemment dosées en trétinoïne, respectivement $10^{-6}$ ; $5.10^{-6}$ ; $10^{-5}$ ; $5.10^{-5}$; $10^{-4}$ ; $10^{-3}$ ; $3.10^{-3}$.

On prépare des solutions de même concentration en trétinoïne, dans de l'alcool à 95° contenant 5% de polyéthylène glycol 400.

On prépare également des solutions témoins, une première solution témoin ne contenant que des liposomes à 3% en lécithine sans trétinoïne, une deuxième composition ne contenant que de l'alcool à 95° avec 5% de polyéthylène glycol 400.

Chacune de ces compositions est appliquée journellement pendant 3 semaines sur la zone scapulaire des souris hairless Rhino à la dose de 0,1ml.

A la fin de cette période, les animaux sont sacrifiés et des biopsies sont effectuées sur la zone d'application. Un examen de microscopie optique couplé à un analyseur d'image, permet de quantifier les coupes par la mesure du diamètre du comédon (D) et de son orifice (d). Cette méthode de mesure de l'activité comédolytique est conforme à celle décrite par Claude BONNE et al. dans International Journal of Cosmetic Science 3,23-28 (1981).

Ainsi, pour chaque animal, la moyenne du rapport $r = d/D$ permet de caractériser l'activité comédolytique de la composition. On procède de manière habituelle en utilisant une composition conforme à l'invention contenant de la trétinoïne dans les liposomes (composition L) en comparaison avec une composition de comparaison selon laquelle la trétinoïne est contenue dans une solution alcoolique (composition A). A titre de solution témoin on utilise une solution témoin contenant des liposomes à 3% de lécithine. Les résultats obtenus font l'objet des courbes A (composition A) et L (composition L) de la figure annexée, ainsi que du tableau I ci-dessous.

Pour obtenir le rapport r = d/D précité la procédure a été modifiée par rapport à celle décrite par Claude BONNE en comptant tous les comédons existant ou potentiels pour une longueur donnée de l'épiderme. Ainsi, on fait la moyenne de tous les d/D de chaque comédon en incluant la valeur d/D = 1 pour les comédons ouverts qui tendent à disparaître à l'observation.

Pour les solutions témoins, on n'observe pas d'activité comédolytique significative.

## TABLEAU I
## ACTIVITE COMEDOLYTIQUE

| Concentration VITAMINE A Acide (trétinoïne) | dans Liposome r=d/D | dans l'alcool à 95° + 55% PEG 400 r=d/D |
|---|---|---|
| témoin | 0,58 | 0,64 |
| $10^{-6}$ | 0,64 | |
| $5.10^{-6}$ | 0,88 | |
| $10^{-5}$ | 0,95 | 0,67 |
| $5.10^{-5}$ | | 0,85 |
| $10^{-4}$ | 0,98 | 0,93 |

PEG = Polyéthylène glycol

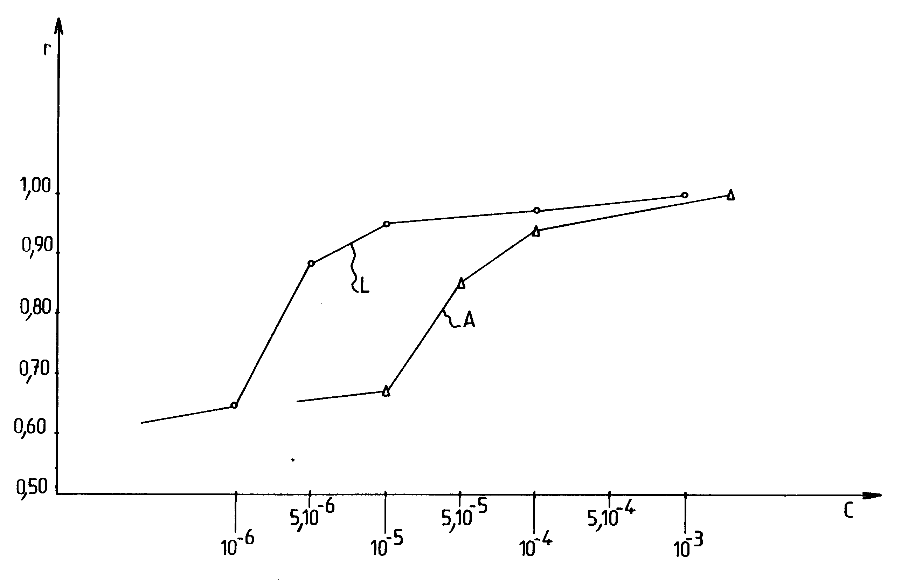

A partir des courbes de la figure 1, on peut observer que la dose $10^{-5}$ de la trétinoïne contenue dans des liposomes entraîne une activité comédolytique maximale qui n'est atteinte qu'à la dose $10^{-4}$ de trétinoïne en solution alcoolique. La formulation d e la trétinoïne dans les liposomes ou phases lamellaires lipidiques hydratées est donc dix fois plus active.

Il est à remarquer que le modèle animal (souris Hairless Rhino) est notablement plus sensible que l'homme, c'est-à-dire que l'activité maximale est atteinte à une concentration plus basse.

D'autre part, le seuil d'efficacité chez la souris hairlesse Rhino est à $5x10^{-6}$ en trétinoïne contenue dans les liposomes selon l'invention alors qu'elle n'est qu'à $5x10^{-5}$ en solvant alcoolique.

Ainsi, l'invention permet de potentialiser l'activité de la trétinoïne en l'incorporant dans des liposomes ou des phases lamellaires lipidiques plus ou moins hydratées.

Nous allons voir maintenant les résultats obtenus en ce qui concerne l'hyperacanthose.

B. Hyperacanthose

On sait que la trétinoïne augmente la mitose des cellules de la membrane basale de l'épiderme, ces cellules montent ensuite vers la couche cornée et produisent l'hyperacanthose.

L'hyperacanthose (H) est déterminée sur les coupes ayant servi à l'évaluation de l'activité comédolytique. On mesure au moyen de l'analyseur d'images l'augmentation H de l'épaisseur e de l'épiderme des animaux traités par rapport aux animaux témoins, la solution témoin étant la solution à base de liposomes à 3% de lécithine.

L'évaluation de l'hypéracanthose est obtenue à partir de la formule suivante :

$$H = \frac{e^t - e^o}{e^o}$$

dans laquelle $e_t$ est l'épaisseur de l'épiderme des animaux traités et $e_o$ celle des animaux témoins.

On obtient les résultats répertoriés au tableau II ci-dessous.

**Tableau II.**

**Hyperacanthose**

| Concentration en trétinoïne / Epaississement | LIPOSOMES | | | ALCOOL | | |
|---|---|---|---|---|---|---|
| | $0$ | $10^{-4}$ | $5\ 10^{-4}$ | $0$ | $10^{-3}$ | $310^{-3}$ |
| $H_1$ (8 jours) | $50,7$ | $88,5$ | $85,3$ | $14,3$ | $59,45$ | $58,1$ |
| $H_2$ (16 jours) | $45$ | $37,7$ | $41,8$ | $19,8$ | $78,8$ | $80,5$ |
| $H = \dfrac{H_1 + H_2}{2}$ | $47,85$ | $63,1$ | $63,55$ | $17,05$ | $69,125$ | $69,3$ |

P.S. : on a répertorié les valeurs obtenues après 8 jours et après 15 jours de traitement, en donnant ensuite la valeur moyenne.

On observe que la trétinoïne contenue dans les liposomes aboutit beaucoup plus rapidement en huit jours seulement, à un épaississement de l'épiderme, ce qui est bénéfique pour le traitement des comédons alors que la trétinoïne en solution alcoolique ne provoque cet effet qu'au bout d'une quinzaine de jours.

D'autre part, on observe également que dans le cas des liposomes, après 16 jours, l'épaississement de l'épiderme diminue, ce qui est également favorable.

## C. L'irritation

L'irritation est étudiée chez le lapin, qui constitue un modèle mieux adapté que la souris Rhino pour ce phénomène.

Une application quotidienne des produits à tester est effectuée à la dose de 0,03 ml/cm$^2$ en patch ouvert sur le flanc droit de chaque lapin d'un groupe de six, le flanc gauche servant de témoin. La durée de traitement est de 2 semaines à raison de 5 jours par semaine. (soit 10 jours de traitement).

On effectue une lecture microscopique chaque jour, conformément au protocole sur les tests d'irritation paru au Journal Officiel en Février 1982. On note l'érythème et l'oedème de 0 à 4.

L'indice I d'irritation est la moyenne des sommes des notes érythème + oedème par jour d'application et par animal, selon la formule suivante :

$$I = \frac{\sum \text{érythème} + \sum \text{oedème}}{60}$$

Lorsque l'indice I d'irritation obtenu est ≤ à 0,5, on considère que la composition ou le produit de traitement est non irritant ;
lorsque I est compris entre 0,5 et 2, on considère que le produit ou la composition est faiblement irritant ;
lorsque I est supérieur à 2 et ≤ à 5, on considère que le produit ou la composition est irritant;
lorsque I est supérieur à 5, on considère que le produit ou la composition est très irritant.

Les résultats obtenus sont répertoriés au tableau III page 22.

On observe que le phénomène d'irritation n'apparait qu'en se plaçant à des concentrations plus élevées que dans l'étude de l'activité comédolytique. Les concentrations testées ont été choisies en fonction des résultats de l'activité comédolytique, en se plaçant donc à un facteur de 10 de différence en concentration pour effectuer une comparaison de l'irritation à activité comédolytique égale, ce qui est déterminant pour le praticien.

On observe que dans ces conditions les deux valeurs de l'irritation obtenues pour les liposomes sont nettement inférieures à celles obtenues pour la solution alcoolique.

Bien entendu, à la différence de l'étude sur l'activité comédolytique où la quantification action-dose est nette et précise, l'évaluation de l'irritation est plus fluctuante et on n'obtient qu'une tendance globale.

On peut donc dire que l'incorporation de la trétinoïne dans les liposomes permet d'obtenir le même résultat sur le traitement des comédons, avec 10 fois moins de produit, sans pour cela augmenter les effets irritants, voire même, elle tend à les diminuer.

De plus, le fait de pouvoir traiter les affections cutanées telles que l'acné avec des doses dix fois plus faibles de trétinoïne diminue d'autant le risque de toxicité de la trétinoïne souvent liée à la dose. En effet, de nombreuses publications décrivent la toxicité de la trétinoïne et de ses homologues structuraux (C.E. Orfanos et al., Hautarzt Juin 1981, 32 (6), pages 275 - 280 ; J.M. Wishart et al. New Zealand Med. J. Oct 28 1981, 94 (694) pages 307-308). Ce sont, outre l'irritation déjà mentionnée : l'hypervitaminose A se traduisant notamment par prurit généralisé, perte de cheveux (C.E. Orfanos déjà cité), des tumeurs osseuses (exostoses) (P.E. Pochi, New England J. Med., Apr. 28 1983, 308 (17) pages 1024 - 1025), la sécheresse des muqueuses, notamment des lèvres (C.E. Orfanos déjà cité), des dermatoses allergiques (C. Romaguera et al., Contact dermatitis Oct. 1980, 6 (6) P. 442) une teratogénicité mise en évidence chez l'animal (L. Newell-Morris et al., Teratology Aug. 1980, 22 (1) p. 87 - 101 ; A.G Hendrickx et al, teratology Aug 1980, 22 (1) pages 13 - 22 ; I.M. Taylor et al, teratology Apr. 1980 21 (2) pages 193 - 7)

Ainsi, les résultats des expérimentations in vivo rapportés ci-dessus permettent de conclure que les compositions selon l'invention dans lesquelles la trétinoïne est incorporée dans des liposomes, ou dans des phases lamellaires lipidiques hydratées, sont utilisables pour préparer des compositions pharmaceutiques ou cosmétiques, particulièrement dans le traitement local de l'acné à des doses situées en dessous du seuil d'activité en milieu alcoolique, tout en ne provoquant pratiquement pas de phénomène d'irritation.

Autrement dit, l'incorporation de la trétinoïne dans des liposomes ou phases lamellaires lipidiques hydratées a abouti à une potentialisation particulièrement inattendue à des doses très faibles de $5.10^{-6}$ à $10^{-4}$, sans potentialiser pour autant le phénomène d'irritation, contrairement à ce qui pouvait être attendu par un homme du métier puisque l'irritation était considérée comme directement liée à l'activité. Ainsi, il est possible de diminuer la dose pour une activité comédolytique équivalente, en abaissant de manière radicale le risque de phénomène d'irritation.

EP 0 229 561 B1

Exemple 3. Composition dermatologique

On prépare selon le mode préféré de l'exemple 1 une suspension de liposomes contenant :

| Trétinoïne | 0,005 g |
|---|---|
| Lécithine de soja hydrogénée | 5 g |
| Sitostérol | 0,5 g |
| $\alpha$ -tocophérol | 0,005 g |

Solution aqueuse tamponnée contenant des antioxydants et des conservateurs, qsp 50g
Ladite suspension est ensuite mélangée à 50 g de gel de Carbopol 940® à 1,25%.
Cette composition finale sous forme de gel est indiquée dans le traitement de l'acné en application quotidienne sur la zone de la peau à traiter.

Exemple 4. Composition dermo-cosmétique

On opère comme dans l'exemple 3, avec la compositon suivante :

| Trétinoïne | 0,001 g |
|---|---|
| Lécithine de soja hydrogénée | 1,0 g |
| Cholestérol | 0,1 g |
| $\alpha$ -Tocophérol | 0,003 g |

Le gel obtenu est utilisé pour le soin des peaux acnéiques.

Exemple 5. Composition dermatologique

A la suspension de liposomes de l'exemple 3, on mélange une quantité égale de gel de Carbopol 940® à 1,25% contenant des conservateurs, des antioxydants et un filtre ultra-violet A + B , aux concentrations habituelles.
Ce gel peut être employé pour le traitement de l'élastose solaire.

Exemple 6. Composition dermo-cosmétique

On réalise une suspension de liposomes selon l'exemple 1, contenant :

| Trétinoïne | 0,002 g |
|---|---|
| Lécithine de soja hydrogénée | 10,00 g |
| Sitostérol | 1,4 g |
| $\alpha$ -tocophérol | 0,06 g |
| "Tampon phosphate" contenant antioxydants et conservateurs qsp | 100 g |

Cette suspension est mélangée à 125 g de gel de carbopol 940® à 1,25%.
Le gel obtenu est émulsionné avec 50 g d'une phase huileuse à base de perhydrosqualène contenant un filtre ultraviolet A + B et un parfum.
L'émulsion gélifiée obtenue est utilisée pour la régénération de la peau du visage ou du corps, et comme agent de restructuration dermique ou cicatrisant.

Exemple 7. Composition dermatologique lyophilisée

On réalise une suspension de liposomes selon l'exemple 1 de composition suivante :

10

| Trétinoïne | 0,005 g |
|---|---|
| Lécithine de soja hydrogénée | 5 g |
| Sitostérol | 0,5 g |
| B.H.T. | 0,005 g |
| "Tampon phosphate", qsp | 100 g |

à laquelle on ajoute 5 g de glucose dans la phase aqueuse comme adjuvant de lyophilisation.

Cette suspension est lyophilisée.

Au moment de l'emploi, on mélange 1 g du lyophilisat avec 20 ml d'eau distillée stérile, en présence de conservateurs hydrosolubles usuels.

La suspension extemporanée peut être utilisée dans le traitement de l'acné, des hyperkératoses ou de l'élastose solaire.

Exemple 8. Composition dermo-cosmétique lyophilisée

On prépare un lyophilisat selon l'exemple 7 en employant 0,001 g de trétinoïne seulement.

La suspension préparée au moment de l'emploi peut être utilisée pour le traitement des peaux acnéiques, des peaux rêches ou des peaux ridées.

Exemple 9. Composition dermatologique

On prépare selon l'exemple 3 un gel de composition suivante :

| Trétinoïne | 0,005 g |
|---|---|
| Lécithine de soja | 5,0 g |
| Sitostérol | 0,5 g |
| Erythromycine | 1,0 g |
| $\alpha$ -tocophérol | 0,003 g |
| Excipients pour gel, qsp | 100 g |

On notera que, dans le cas présent, l'érythromycine est introduite dans la solution organique de dichlorométhane, avant l'atomisation.

Le gel obtenu est utilisé dans le traitement de l'acné.

Exemple 10. Composition dermatologique

On prépare selon l'exemple 1 une poudre lipidique, atomisée contenant :

| Trétinoïne | 0,005 g |
|---|---|
| Lécithine | 1,0 g |
| Sitostérol | 0,1 g |
| $\alpha$ -tocophérol | 0,003 g |

Celle-ci est dispersée dans un "tampon phosphate" contenant 1% de chlorhydrate de clindamycine, ainsi que des antioxydants, qsp 100 g.

On obtient une suspension de liposomes qui contiennent du chlorhydrate de clindamycine dans la phase aqueuse encapsulée.

Cette suspension peut être lyophilisée, dans ce cas, on ajoute préalablement 100 ml d'une solution aqueuse de glucose à 10%.

Au moment de l'emploi, on mélange 1g du lyophilisat avec 20 ml d'eau distillée stérile, en présence de conservateurs hydrosolubles usuels.

La suspension extemporanée est utilisée avantageusement dans le traitement de l'acné.

Exemple 11. Composition dermatologique

On prépare un gel selon l'exemple 3, toutefois la solution aqueuse tamponnée contient de l'hydroquinone.

La composition de la suspension de liposomes, avant mélange avec le gel de Carbopol 940® est la suivante:

| | |
|---|---|
| Trétinoïne | 0,005 g |
| Lécithine de soja hydrogénée | 5,0 g |
| Sitostérol | 0,5 g |
| Antioxydant liposoluble | 0,2 g |
| Hydroquinone | 3,0 g |
| Sulfite de sodium | 0,4 g |
| Acide ascorbique | 0,5 g |
| "Tampon phosphate" qsp | 50 g |

Le gel sera utilisé en application topique pour le traitement des taches pigmentaires.

Exemple 12. Composition dermo-cosmétique

On opère selon l'exemple 11, mais en diminuant les proportions de trétinoïne et d'hydroquinone.
La composition finale du gel est la suivante:

| | |
|---|---|
| Trétinoïne | 0,001 g |
| Lécithine de soja hydrogénée | 5,0 g |
| Sitostérol | 0,5 g |
| Antioxydant liposoluble | 0,2 g |
| Hydroquinone | 2,0 g |
| Sulfite de sodium | 0,4 g |
| Acide ascorbique | 0,5 g |
| Gel de Carbopol 940® à 1,25% qsp | 100 g |

Ce gel est utilisé en application sur les taches pigmentaires de la peau, telles que les taches dites de vieillesse, pour les faire disparaître ou tout au moins les atténuer.

Exemple 13. Composition dermo-cosmétique

On prépare une suspension de liposomes selon l'exemple 1 de composition suivante :

| | |
|---|---|
| Trétinoïne | 0,0005 g |
| Lécithine de soja | 0,5 g |
| Cholestérol | 0,05 g |
| $\alpha$ -tocophérol | 0,0003 g |
| Excipients aqueux composés d'un "tampon phosphate" de conservateurs, d'antioxydants et d'extraits aqueux parfumés, qsp | 100 ml |

La suspension obtenue est une lotion utilisée pour favoriser la pousse des cheveux.

EP 0 229 561 B1

TABLEAU III.

IRRITATION DE LA PEAU DE LAPIN APRES 15 JOURS DE TRAITEMENT

| Composition | concentration en trétinoïne | Valeur moyenne d'irritation | | | | | |
|---|---|---|---|---|---|---|---|
| | témoin (0) | $5.10^{-6}$ | $5.10^{-5}$ | $10^{-4}$ | $5.10^{-4}$ | $10^{-3}$ | $3.10^{-3}$ |
| dans liposomes (invention) | 2.34 | NS | NS | 2.67 | 3.24 | | |
| dans l'alcool +PEG (comparatif) 400 5% | 1.08 | NS | NS | | | 3.13 | 3.95 |

N.S. = Non Significatif

Exemple 14. Composition dermatologique.

On prépare par atomisation, selon le mode préféré de l'exemple 1, une poudre A de composition suivante :

13

| Trétinoïne | 0,05 g |
|---|---|
| Lécithine de soja hydrogénée | 20,0 g |
| Stéarate d'érythromycine | 10,0 g |
| Alpha-tocophérol | 0,05 g |

On prépare d'autre part, une solution aqueuse B de composition suivante :

| Acétate d'ascorbyle | 0,01 g |
|---|---|
| E.D.T.A. | 0,01 g |
| Saccharose | 5,0 g |
| Glycine | 2,5 g |
| Eau q.s.p. | 100,0 g |

1 g de la poudre A est ensuite dispersée dans 100 g de solution B sous agitation magnétique pendant 2 heures à température ambiante.

Il se forme une suspension de liposomes qui est ensuite homogénéisée avec ultra-sons à 4°C pendant 10 minutes à la puissance de 200W. La taille moyenne des liposomes après cette homogénéisation est de 200 nanomètres.

La suspension de liposomes obtenue est ensuite lyophilisée à -40°C pour produire une poudre, qui peut être conservée en récipient fermé pendant plusieurs années.

En vue de son utilisation en dermatologie, cette poudre lyophilisée est agitée en présence d'un milieu aqueux approprié tel qu'un gel de Carbopol 940® de bonne viscosité (100 à 1000 centipoises environ). La proportion de poudre lyophilisée dans le gel est par exemple de 1%.

Ce gel obtenu de façon extemporanée, peut être utilisé en application quotidienne sur la peau pour le traitement de l'acné.

Exemple 15. Composition dermo-cosmétique.

On prépare un gel selon l'exemple 3 avec la composition suivante :

| Acétate de vitamine A | 0,2 g |
|---|---|
| Lécithine de soja hydrogénée | 1,8 g |
| B.H.T. | 0,02g |
| Excipient pour gel, q.s.p. | 100,0 g |

Dans le présent exemple, l'acétate de vitamine A et le B.H.T. sont introduits dans la solution de dichlorométhane avant l'atomisation.

Le gel obtenu en application sur la peau, favorise le renouvellement de l'épiderme.

Exemple 16. Composition dermo-cosmétique.

On prépare un gel selon l'exemple 15, mais en remplaçant les 0,2 g d'acétate de vitamine A par 0,4 g de palmitate de vitamine A. La quantité de lécithine est de 1,6 g seulement.

Ce gel favorise également le renouvellement de l'épiderme.

Exemple 17. Composition dermo-cosmétique.

On prépare un gel selon l'exemple 3 avec la composition suivante.

| Béta-carotène | 0,2 g |
|---|---|
| Lécithine de soja | 1,8 g |
| Alpha-tocophérol | 0,002 g |
| Excipient pour gel, q.s.p. | 100,0 g |

Le gel obtenu est émulsionné avec 100 g d'une phase huileuse à base de perhydrosqualène contenant du parfum.

L'émulsion gélifiée obtenue est utilisée pour préparer la peau avant l'exposition au soleil.

## Revendications

1. Utilisation à titre de produit cosmétique d'une composition à base de phases lamellaires lipidiques hydratées ou de liposomes contenant à titre d'agent actif un rétinoïde ou un caroténoïde, destinée au traitement des affections cutanées, des troubles de la kératinisation, de l'élastose solaire, des comédons, des peaux acnéiques, des peaux rêches, des peaux ridées, à la lutte contre le vieillissement cutané actinique ou chronologique, à l'atténustion ou à la suppression des tâches pigmentaires cutanées, notamment des tâches dites de vieillesse, à favoriser la cicatrisation, à la reconstitution des micro-vaisseaux sanguins, notamment dans le derme, à stimuler la pousse des cheveux, à régénérer la peau du visage ou du corps, à la restructuration dermique, à favoriser le renouvellement ou l'épaississement de l'épiderme, ou à préparer la peau avant l'exposition au soleil.

2. Utilisation selon la revendication 1, caractérisée en ce que le rétinoïde précité est choisi parmi la trétinoïne ou ses dérivés, notamment, sels ou esters ; l'acide 13-cis-rétinoïque ou ses dérivés notamment, sels ou esters, la vitamine A ou ses esters, l'étrétinate.

3. Utilisation selon la revendication 1, caractérisée en ce que le caroténoïde précité est choisi parmi l'$\alpha$- ou $\beta$-carotène; ou le $\gamma$-carotène ou le $\delta$-carotène.

4. Utilisation selon la revendication 1 ou 2, caractérisée en ce que le rétinoïde précité est la trétinoïne.

5. Utilisation selon la revendication 4, caractérisée en ce que la concentration en trétinoïne par rapport à la composition totale est de $10^{-6}$ à $10^{-3}$.

6. Utilisation selon la revendication 4, caractérisée en ce que la concentration en trétinoïne par rapport à la composition totale est de $5.10^{-6}$ à $10^{-4}$.

7. Utilisation selon l'une des revendications 1 à 6, caractérisée en ce que la proportion du rétinoïde ou du caroténoïde dans la phase lipidique constituant la bicouche de la phase lamellaire lipidique précitée ou du liposome, peut atteindre 40% en poids, de préférence est comprise entre $10^{-4}$% et 10% et encore de préférence est de 0,01% à 5%.

8. Utilisation selon l'une des revendications précédentes, caractérisée en ce que les phases lamellaires lipidiques hydratées ou les liposomes contiennent en outre des substances hydrophobes telles que des stérols, comme le sistostérol, le cholestérol, le stigmastérol.

9. Utilisation selon l'une quelconque des revendications 1 à 8, caractérisée en ce que la phase lipidique de la phase lamellaire lipidique hydratée précitée ou du liposome précité contient un antioxydant tel que l'$\alpha$-tocophérol jusqu'au plus 1% en poids, du B.H.T. ou du T.B.H.Q.; et/ou la phase aqueuse contient un antioxydant hydrosoluble tel que l'acide ascorbique, le sulfite de sodium, le bisulfite de sodium, l'EDTA, avantageusement aussi des stabilisants tels que l'acide citrique, l'histidine.

10. Utilisation selon l'une quelconque des revendications 1 à 9, caractérisée en ce que la composition précitée contient de préférence un composé constituant un filtre ultraviolet.

11. Utilisation selon l'une quelconque des revendications 1 à 10, caractérisée en ce que la composition précitée est préparée à partir d'une poudre lipidique lyophilisée.

12. Utilisation d'une composition à base de phases lamellaires lipidiques hydratées ou de liposomes contenant à titre d'agent actif un rétinoïde ou un caroténoïde pour la fabrication d'un médicament destiné au traitement par application topique des affections cutanées, telles que les différentes formes d'acné, de l'hyperkératose, du psoriasis ou autres troubles de la kératinisation, de l'élastose solaire, des comédons, des peaux acnéiques, des peaux rêches, des peaux ridées, à la lutte contre le vieillissement cutané actinique ou chronologique, à l'atténuation ou à la suppression des tâches pigmentaires

EP 0 229 561 B1

cutanées, notamment des tâches dites de vieillesse, à favoriser la cicatrisation, à la reconstitution des micro-vaisseaux sanguins, notamment dans le derme, à stimuler la pousse des cheveux, à régénérer la peau du visage ou du corps, à la restructuration dermique, à favoriser le renouvellement ou l'épaississement de l'épiderme et à préparer la peau avant l'exposition au soleil, ou destiné, par administration orale, en particulier au traitement des différentes formes d'acné, de l'hyperkératose et du psoriasis.

13. Utilisation selon la revendication 12, caractérisée en ce que le rétinoïde précité est choisi parmi la trétinoïne ou sec dérivés, notamment, sels ou esters ; l'acide 13-cis-rétinoïque ou ses dérivés notamment, sels ou esters, la vitamine A ou ses esters, l'étrétinate.

14. Utilisation selon la revendication 12, caractérisée en ce que le caroténoïde précité est choisi parmi l'$\alpha$- ou $\beta$-carotène; ou le $\gamma$-carotène ou le $\delta$-carotène.

15. Utilisation selon la revendication 12 ou 13, caractérisée en ce que le rétinoïde précité est la trétinoïne.

16. Utilisation selon la revendication 15, caractérisée en ce que la concentration en trétinoïne par rapport à la composition totale est de $10^{-6}$ à $10^{-3}$.

17. Utilisation selon la revendication 15, caractérisée en ce que la concentration en trétinoïne par rapport à la composition totale est de $5.10^{-6}$ à $10^{-4}$.

18. Utilisation selon l'une des revendications 12 à 17, caractérisée en ce que la proportion du rétinoïde ou du caroténoïde dans la phase lipidique constituant la bicouche de la phase lamellaire lipidique précitée ou du liposome, peut atteindre 40% en poids, de préférence est comprise entre $10^{-4}$% et 10% et encore de préférence est de 0,01% à 5%.

19. Utilisation selon l'une des revendications 12 à 18, caractérisée en ce que les phases lamellaires lipidiques hydratées précitées ou les liposomes précités contiennent en outre un agent antibiotique ou antiseptique, tel que l'érythromycine, la clindamycine, le métronidazole, ou leurs dérivés, notamment les esters.

20. Utilisation selon la revendication 19, caractérisée en ce que les phases lamellaires lipidiques hydratées précitées ou les liposomes précités contiennent la trétinoïne ou l'un de ses dérivés notamment, sels ou esters, en combinaison avec l'érythromycine ou l'un de ses dérivés dont la proportion peut atteindre 4% en poids par rapport à la composition totale, et se situe préférentiellement à 1%.

21. Utilisation selon la revendication 19, caractérisée en ce que les phases lamellaires lipidiques hydratées ou les liposomes précités contiennent la trétinoïne ou l'un de ses dérivés notamment, sels ou esters, en combinaison avec la clindamycine ou l'un de ses dérivés dont la proportion peut atteindre 2% en poids par rapport à la composition totale.

22. Utilisation selon l'une des revendications 12 à 21, caractérisée en ce que les phases lamellaires lipidiques hydratées ou les liposomes contiennent en outre des substances hydrophobes telles que des stérols, comme le sistostérol, le cholestétol, le stigmastérol.

23. Utilisation selon l'une quelconque des revendications 12 à 22, caractérisée en ce que la phase lipidique de la phase lamellaire lipidique hydratée précitée on du liposome précité contient un antioxydant tel que l'$\alpha$-tocophérol jusqu'au plus 1% en poids, du B.H.T. ou du T.B.H.Q.; et/ou la phase aqueuse contient un antioxydant hydrosoluble tel que l'acide ascorbique, le sulfite de sodium, le bisulfite de sodium, l'EDTA, avantageusement aussi des stabilisants tels que l'acide citrique, l'histidine.

24. Utilisation selon l'une quelconque des revendications 12 à 23, caractérisée en ce que la composition précitée contient de préférence un composé constituant un filtre ultraviolet.

25. Utilisation selon l'une quelconque dos revendications 12 à 24, caractérisée en ce que la composition précitée est préparée à partir d'une poudre lipidique lyophilisée.

**Claims**

16

1. Utilization as a cosmetic product of a composition based upon hydrous lipidic lamellar phases or liposomes containing as an active agent a retinoid or a carotenoid intended for the treatment of skin diseases, carotenization troubles, solar elastosis, comedos, acneic skins, harsh skins, wrinkled skins, for the fight against actinic or chronological skin ageing, for the attenuation or the removal of pigmental skin spots, in particular of the so-called senile spots, for promoting the cicatrization, for the regeneration of blood micro-vessels in particular in the derm; for stimulating hair growth, for regenerating the skin of the face or of the body, for the dermic restructuring, for promoting the renewal or the thickening of the epiderm, or for preparing the skin prior to the exposure to the sun.

2. Utilization according to claim 1, characterized in that the aforesaid retinoid is selected among tretinoin or its derivatives, in particular salts or esters; the 13-cis-retinoic acid or its derivatives, in particular salts or esters, the A vitamin or its esters, the etratinate.

3. Utilization according to claim 1, characterized in that the aforesaid carotenoid is selected among the $\alpha$- or $\beta$-carotene; or the $\gamma$-carotene or the $\delta$-carotene.

4. Utilization according to claim 1 or 2, characterized in that the aforesaid retinoid is the tretinoin.

5. Utilization according to claim 4, characterized in that the tretinoin concentration with respect to the total composition is from $10^{-6}$ to $10^{-3}$.

6. Utilization according to claim 4, characterized in that the tretinoin concentration with respect to the total composition is from $5.10^{-6}$ to $10^{-4}$.

7. Utilization according to one of claims 1 to 6, characterized in that the proportion of retinoid or carotenoid in the lipidic phase constituting the bilayer of the aforesaid lipidic lamellar phase or of the liposome may reach 40% by weight, preferably is comprised between $10^{-4}$ % and 10% or still preferably is from 0.01% to 5%.

8. Utilization according to one of the foregoing claims, characterized in that the hydrous lipidic lamellar phases or the liposomes further contain hydrophobic substances such as sterols, like the sistosterol, the cholesterol, the stigmasterol.

9. Utilization according to any one of claims 1 to 8, characterized in that the lipidic phase of the aforesaid hydrous lipidic lamellar phase or of the aforesaid liposome contains an anti-oxidant such as the $\alpha$-tocopherol up to at most 1% by weight, B.H.T. or T.E.H.Q.; and/or the aqueous phase contains a water-soluble anti-oxidant such as the ascorbic acid, the sodium sulfite, the sodium bisulfite, the EDTA, also advantageously stabilizers such as the citric acid and the histidine.

10. Utilization according to any one of claims 1 to 9, characterized in that the aforesaid composition preferably contains a compound constituting an ultraviolet filter.

11. Utilization according to any one of claims 1 to 10, characterized in that the aforesaid composition is prepared from a lyophilised lipidic powder.

12. Utilization of a composition based upon hydrous lipidic lamellar phases or on liposomes containing as an active agent a retinoid or a caretonoid for the manufacture of a medicine intended for the treatment, by a topical application, of skin diseases such as various forms of acne, hyperkeratosis, psoriasis or other troubles of the keratinization, solar elastosis, comedos, acneic skins, harsh skins, wrinkled skins, for the fight against actinic or chronological skin ageing, for the attenuation or the removal of pigmental skin spots, in particular of the so-called senile spots, for promoting the cicatrization, for the reconstitution of blood microvessels, in particular in the derm, for stimulating hair growth, for regenerating the skin of the face or of the body, for the dermic restructuring, for promoting the renewal or the thickening of the epiderm and for preparing the skin before exposure to the sun, or intended for oral administration, in particular for the treatment of various forms of acne, of the hyperkeratosis and of the psoriasis.

13. Utilization according to claim 12, characterized in that the aforesaid retinoid is selected among tretinoin

17

or its derivatives, in particular salts or esters; the 13-cis-retinoic acid or its derivatives, in particular salts or esters, the A vitamin or its esters, the etretinate.

**14.** Utilization according to claim 12, characterized in that the aforesaid carotenoid is selected among the $\alpha$- or $\beta$-carotene; or the $\gamma$-carotene or the $\delta$-carotene.

**15.** Utilization according to claim 12 or 13, characterized in that the aforesaid retinoid is the tretinoin.

**16.** Utilization according to claim 15, characterized in that the tretinoin concentration with respect to the total composition is from $10^{-6}$ to $10^{-3}$.

**17.** Utilization according to claim 15, characterized in that the tretinoin concentration with respect to the total composition is from $5.10^{-6}$ to $10^{-4}$.

**18.** Utilization according to one of claims 12 to 17, characterized in that the proportion of the retinoid or of the carotenoid in the lipidic phase constituting the bilayer of the aforesaid lipidic lamellar phase or of the liposome may reach 40% by weight, preferably is comprised between $10^{-4}$% and 10% and still preferably is from 0.01% to 5%.

**19.** Utilization according to one of claims 12 to 18, characterized in that the aforesaid hydrous lipidic lamellar phases or the aforesaid liposomes further contain an antibiotic or antiseptic agent such as the erythromycin, the clindamycin, the metronidazole or their derivatives, in particular the esters.

**20.** Utilization according to claim 19, characterized in that the aforesaid hydrous lipidic lamellar phases or the aforesaid liposomes contain the tretinoin or one of its derivatives, in particular salts or esters, in combination with the erythromycin or one of its derivatives the proportion of which may reach 4% by weight with respect to the total composition and is lying preferably at 1%.

**21.** Utilization according to claim 19, characterized in that the aforesaid hydrous lipidic lamellar phases or the aforesaid liposomes contain the tretinoin or one of its derivatives, in particular salts or esters, in combination with the clindamycin or one of its derivatives the proportion of which may reach 2% by weight with respect to the total composition.

**22.** Utilization according to one of claims 12 to 21, characterized in that the hydrous lipidic lamellar phases or the liposomes further contain hydrophobic substances such as sterols, like the sistosterol, the cholesterol, the stigmasterol.

**23.** Utilization according to any one of claims 12 to 22, characterized in that the lipidic phase of the aforesaid hydrous lipidic lamellar phase or of the aforesaid liposome contains an anti-oxidant such as the $\alpha$-tocopherol up to at most 1% by weight, B.H.T. or T.E.H.Q.; and/or the aqueous phase contains a water-soluble anti-oxidant such as the ascorbic acid, the sodium sulfite, the sodium bisulfite, the EDTA, also advantageously stabilizers such as the citric acid and the histidine.

**24.** Utilization according to any one of claims 12 to 23, characterized in that the aforesaid composition preferably contains a compound constituting an ultraviolet filter.

**25.** Utilization according to any one of claims 12 to 24, characterized in that the aforesaid composition is prepared from a lyophilised lipidic powder.

## Patentansprüche

**1.** Verwendung als ein kosmetisches Erzeugnis einer Zusammensetzung auf Basis von wasserhaltigen, lipidischen, lamellaren Phasen oder von Liposomen, die ein Retinoid oder ein Karotinoid als Wirkstoff enthalten, und zur Behandlung von Hautkrankheiten, von. Verhornungprozessstörungen, der Sonnenelastosis, der Mitesser, der akneischen Häute, der rauhen Häute, der runzligen Häute, zur Bekämpfung des aktinischen oder chronologischen Hautalterns, zur Verminderung oder Unterdrückung der Hautpigmentflecken, insbesondere der sogenannten Altersflecken, zur Begünstigung der Vernarbung, zur Wiederherstellung der Mikroblutgefässe, insbesondere in der äusseren Haut, zur Anregung des Haarwuchses, zur Regeneration der Gesichts-oder der Körperhaut, zum Wiederstrukturieren der Lederhaut, und zur Begünstigung der Erneuerung oder der Verdickung der Epidermis oder für die Vorbereitung der Haut vor der Aussetzung in die Sonne, bestimmt ist.

2. Verwendung nach Anspruch 1, dadurch gekennzeichnet, dass das vorgenannte Retinoid unter Tretinoin oder dessen Derivaten, insbesondere Salzen oder Estern; 13-cis-Retinoïnsäure oder deren Derivaten, insbesondere Salzen oder Estern, Vitamin A oder deren Estern, Etretinat, gewählt wird.

3. Verwendung nach Anspruch 1, dadurch gekennzeichnet, dass das vorgenannte Karotinoid unter $\alpha$-oder $\beta$-Karotin; oder $\gamma$-Karotin oder $\delta$-Karotin gewählt wird.

4. Verwendung nach Anspruch 1 oder 2, dadurch gekennzeichnet, dass das vorgenannte Retinoid Tretinoin ist.

5. Verwendung nach Anspruch 4, dadurch gekennzeichnet, dass die Konzentration an Tretinoin in Bezug auf die gesamte Zusammensetzung $10^{-6}$ bis $10^{-3}$ beträgt.

6. Verwendung nach Anspruch 4, dadurch gekennzeichnet, dass die Konzentration an Tretinoin in Bezug auf die gesamte Zusammensetzung $5.10^{-6}$ bis $10^{-4}$ beträgt.

7. Verwendung nach einem der Ansprüche 1 bis 6, dadurch gekennzeichnet, dass das Verhältnis des Retinoids oder des Karotinoids in der die Doppelschicht der vorgenannten lipidischen lamellaren Phase oder des Liposoms bildenden lipidischen Phase 40 Gewichtprozent erreichen kann, vorzugsweise zwischen $10^{-4}$ % und 10 % liegt und noch vorzugsweise von 0,01 % bis 5 % beträgt.

8. Verwendung nach einem der vorangehenden Ansprüche, dadurch gekennzeichnet, dass die wasserhaltigen lipidischen lamellaren Phasen oder die Liposomen ausserdem hydrophobe Substanzen wie Sterine, wie Sistosterin, Cholesterin, Stigmasterin enthalten.

9. Verwendung nach irgendeinem der Ansprüche 1 bis 8, dadurch gekennzeichnet, dass die lipidische Phase der vorgenannten wasserhaltigen lipidischen lamellaren Phase oder des vorgenannten Liposoms einen Oxidationsinhibitor, wie $\alpha$-Tocopherol bis höchstens 1 Gewichtsprozent, B.H.T. oder T.B.H.Q. enthält; und/oder dass die wässerige Phase einen wasserlöslichen Oxidationsinhibitor wie Ascorbinsäure, Natriumsulfit, Natriumbisulfit, EDTA, vorteilhaft auch Stabilisierungsmittel wie Zitronensäure, Histidin enthält.

10. Verwendung nach irgendeinem der Ansprüche 1 bis 9, dadurch gekennzeichnet, dass die vorgenannte Zusammensetzung vorzugsweise eine einen Ultraviolett-Filter bildende Verbindung enthält.

11. Verwendung nach irgendeinem der Ansprüche 1 bis 10, dadurch gekennzeichnet, dass die vorgenannte Zusammensetzung aus einem lyophilisierten lipidischen Pulver zubereitet wird.

12. Verwendung einer Zusammensetzung auf Basis von wasserhaltigen lipidischen lamellaren Phasen oder von Liposomen, die ein Retinoid oder ein Karotinoid als Wirkstoff enthalten, zur Herstellung eines Arzneimittels, dass zur Behandlung durch topisches Aufbringen von Hautkrankheiten, wie verschiedene Formen von Akne, der Hyperkeratosis, der Psoriasis, oder von anderen Störungen des Verhornungsprozesses, der Sonnenelastosis, der Mitesser, der akneischen Häute, der rauhen Häute, der runzligen Häute, zur Bekämpfung des aktinischen oder chronologischen Hautalterns, zur Verminderung oder Unterdrückung der Hautpigmentflecken, insbesondere der sogenannten Altersflecken, zur Begünstigung der Vernarbung, zur Wiederherstellung der Mikroblutgefässe, insbesondere in der Lederhaut, zur Anregung des Haarwuchses, zur Regenerierung der Gesichts-oder der Körperhaut, zur äusseren Hautrestrukturierung, zur Begünstigung der Erneuerung oder der Verdickung der Oberhaut und zur Vorbereitung der Haut vor der Aussetzung in die Sonne bestimmt ist oder über orale Verabreichung,insbesondere zur Behandlung der verschiedenen Formen von Akne, der Hyperkeratosis und der Psoriasis bestimmt ist.

13. Verwendung nach Anspruch 12, dadurch gekennzeichnet, dass das vorgenannte Retinoid unter Tretinoin oder dessen Derivaten, insbesondere Salzen oder Estern; 13-cis-Retinoinsäure oder deren Derivaten, insbesondere Salzen oder Estern, Vitamin A oder deren Estern, Etretinat gewählt wird.

**14.** Verwendung nach Anspruch 12, dadurch gekennzeichnet, dass das vorgenannte Karotinoid unter $\alpha$- oder $\beta$-Karotin; oder $\gamma$-Karotin oder $\delta$-Karotin gewählt wird.

**15.** Verwendung nach Anspruch 12 oder 13, dadurch gekennzeichnet, dass das vorgenannte Retinoid das Tretinoin ist.

**16.** Verwendung nach Anspruch 15, dadurch gekennzeichnet, dass die Konzentration an Tretinoin in Bezug auf die gesamte Zusammensetzung $10^{-6}$ bis $10^{-3}$ beträgt.

**17.** Verwendung nach Anspruch 15, dadurch gekennzeichnet, dass die Konzentration an Tretinoin in Bezug auf die gesamte Zusammensetzung $5.10^{-6}$ bis $10^{-4}$ beträgt.

**18.** Verwendung nach einem der Ansprüche 12 bis 17, dadurch gekennzeichnet, dass das Verhältnis des Retinoids oder des Karotinoids in der die Doppelschicht der vorgenannten lipidischen lamellaren Phase bildenden lipidischen Phase oder des Liposoms 40 Gewichtsprozent erreichen kann, vorzugsweise zwischen $10^{-4}$ % und 10 % liegt oder noch vorzugsweise 0,01% bis 5% beträgt.

**19.** Verwendung nach einem der Ansprüche 12 bis 18, dadurch gekennzeichnet, dass die vorgenannten wasserhaltigen lipidischen lamellaren Phasen oder die vorgenannten Liposomen ausserdem ein antibiotisches oder keimtötendes Mittel wie Erythromycin, Clindamycin, Metronidazol oder deren Derivaten, insbesondere Estern enthalten.

**20.** Verwendung nach Anspruch 19, dadurch gekennzeichnet, dass die vorgenannten wasserhaltigen lipidischen lamellaren Phasen oder die vorgenannten Liposomen Tretinoin oder einen dessen Derivaten, insbesondere Salzen oder Estern enthalten, in Verbindung mit Erythromycin oder einem dessen Derivaten, dessen Verhältnis 4 Gewichtsprozent in Bezug auf die gesamte Zusammensetzung erreichen kann und vorzugsweise bei 1% liegt.

**21.** Verwendung nach Anspruch 19, dadurch gekennzeichnet, dass die vorgenannten wasserhaltigen lipidischen lamellaren Phasen oder Liposomen Tretinoin oder eines dessen Derivaten, insbesondere Salzen oder Estern enthalten, in Verbindung mit Clindamycin oder einem dessen Derivaten, dessen Verhältnis 2 Gewichtsprozent in Bezug auf die gesamte Zusammensetzung erreichen kann.

**22.** Verwendung nach einem der Ansprüche 12 bis 21, dadurch gekennzeichnet, dass die wasserhaltigen lipidischen lamellaren Phasen oder die Liposomen ausserdem hydrophobe Substanzen wie Sterine, wie Sistosterin, Cholesterin, Stigmasterin enthalten.

**23.** Verwendung nach irgendeinem der Ansprüche 12 bis 22, dadurch gekennzeichnet, dass die lipidische Phase der vorgenannten wasserhaltigen lipidischen lamellaren Phase oder des vorgenannten Liposoms einen Oxidationsinhibitor, wie $\alpha$-Tocopherol bis höchstens 1 Gewichtsprozent, B.H.T. oder T.B.H.Q. enthält; und/oder dass die wässerige Phase einen wasserlöslichen Oxidationsinhibitor wie Ascorbinsäure, Natriumsulfit, Natriumbisulfit, EDTA, vorteilhaft auch Stabilisierungsmittel wie Zitronensäure, Histidin enthält.

**24.** Verwendung nach irgendeinem der Ansprüche 12 bis 23, dadurch gekennzeichnet, dass die vorgenannte Zusammensetzung vorzugsweise eine einen Ultraviolett-Filter bildende Verbindung enthält.

**25.** Verwendung nach irgendeinem der Ansprüche 12 bis 24, dadurch gekennzeichnet, dass die vorgenannte Zusammensetzung aus einem lyophilisierten lipidischen Pulver zubereitet wird.